(19) Europäisches Patentamt / European Patent Office / Office européen des brevets

(11) **EP 1 549 432 B1**

(12) **EUROPEAN PATENT SPECIFICATION**

(45) Date of publication and mention
of the grant of the patent:
**24.08.2011 Bulletin 2011/34**

(51) Int Cl.:
*B01J 23/68* [(2006.01)]     *C07C 51/25* [(2006.01)]
*C07C 51/215* [(2006.01)]

(21) Application number: **03748285.8**

(22) Date of filing: **23.09.2003**

(86) International application number:
**PCT/GB2003/004060**

(87) International publication number:
**WO 2004/033090 (22.04.2004 Gazette 2004/17)**

(54) **MIXED METAL OXIDE CATALYST AND PROCESS FOR PRODUCTION OF ACETIC ACID**

MISCHOXIDKATALYSATOR UND VERFAHREN ZUR HERSTELLUNG VON ESSIGSÄURE

CATALYSEUR COMPOSE D'OXYDE DE METAUX MELANGES ET PROCEDE DE PRODUCTION D'ACIDE ACETIQUE

(84) Designated Contracting States:
**AT BE BG CH CY CZ DE DK EE ES FI FR GB GR HU IE IT LI LU MC NL PT RO SE SI SK TR**

(30) Priority: **10.10.2002 GB 0223681**

(43) Date of publication of application:
**06.07.2005 Bulletin 2005/27**

(73) Proprietor: **BP Chemicals Limited Sunbury-on-Thames, Middlesex TW16 7BP (GB)**

(72) Inventor: **ELLIS, Brian Cottingham East Yorkshire HU16 4HY (GB)**

(74) Representative: **Brooke, Caron BP International Limited Patents & Agreements Chertsey Road Sunbury-on-Thames, Middlesex TW16 7LN (GB)**

(56) References cited:
**EP-A- 1 043 064     EP-A- 1 192 987**

• **RUTH K ET AL: "Mo-V-Nb Oxide Catalysts for the Partial Oxidation of Ethane - II. Chemical and Catalytic Properties and Structure Function Relationships" JOURNAL OF CATALYSIS, ACADEMIC PRESS, DULUTH, MN, US, vol. 175, no. 1, 1 April 1998 (1998-04-01), pages 27-39, XP004465689 ISSN: 0021-9517**

Remarks:
The file contains technical information submitted after the application was filed and not included in this specification

**Description**

The present invention relates to a catalyst for the selective oxidation of ethane to acetic acid and/or for the selective oxidation of ethylene to acetic acid, and to a process for the production of acetic acid utilizing the aforesaid catalyst.

Catalysts comprising molybdenum, vanadium and niobium in combination with oxygen for use in processes for the production of acetic acid by the oxidation of ethane and ethylene are known in the art from, for example, US 4,250,346; EP-A-1043064, WO 99/20592 and DE 196 30 832.

US Patent No. 4,250,346 discloses the oxidative dehydrogenation of ethane to ethylene in a gas phase reaction at relatively high levels of conversion, selectivity and productivity to ethylene at a temperature of less than about 550°C using as a catalyst a composition comprising the elements molybdenum, X and Y in the ratio $Mo_aX_bY_c$ wherein X is Cr, Mn, Nb, Ta, Ti, V and/or W, and preferably Mn, Nb, V and/or W; Y is Bi, Ce, Co, Cu, Fe, K, Mg, Ni, P, Pb, Sb, Si, Sn, Tl and/or U, and preferably Sb, Ce and/or U, a is 1, b is 0.05 to 1.0 and c is 0 to 2, and preferably 0.05 to 1.0, with the proviso that the total value of c for Co, Ni and/or Fe is less than 0.5.

WO 99/20592 relates to a method of selectively producing acetic acid from ethane, ethylene or mixtures thereof and oxygen at high temperature in the presence of a catalyst having the formula $Mo_aPd_bX_cY_d$ wherein X represents one or several of Cr, Mn, Nb, Ta, Ti, V, Te and W; Y represents one or several of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, T1 and U and a=1, b=0.0001 to 0.01, c = 0.4 to 1 and d = 0:005 to 1.

German patent application DE 196 30 832 A1 relates to a similar catalyst composition in which a =1, b > 0, c > 0 and d = 0 to 2. Preferably, a = 1, b = 0.0001 to 0.5, c = 0.1 to 1.0 and d = 0 to 1.0.

The catalysts of both WO 99/20592 and DE 19630832 require the presence of palladium.

EP-A-1043064 discloses a catalyst composition for the oxidation of ethane to ethylene and/or acetic acid and/or for the oxidation of ethylene to acetic acid which comprises in combination with oxygen the elements molybdenum, vanadium, niobium and gold in the absence of palladium according to the empirical formula :

$$Mo_aW_bAu_cV_dNb_eY_f \text{ (I)}$$

wherein Y is one or more elements selected from the group consisting of : Cr, Mn, Ta, Ti, B, Al, Ga, In, Pt, Zn, Cd, Bi, Ce, Co, Rh, Ir, Cu, Ag, Fe, Ru, Os, K, Rb, Cs, Mg, Ca, Sr, Ba, Zr, Hf, Ni, P, Pb, Sb, Si, Sn, Tl, U, Re, Te and La; a, b, c, d, e and f represent the gram atom ratios of the elements such that : $0 < a \le 1$; $0 \le b < 1$ and $a + b = 1$; $10^{-5} < c \le 0.02$; $0 < d \le 2$; $0 < e \le 1$; and $0 \le f \le 2$.

There remains a need to develop a catalyst for the oxidation of ethane and/or ethylene to acetic acid and a process for the production of acetic acid using said catalyst and wherein the catalyst enables a high selectivity to acetic acid to be achieved.

Surprisingly, it has now been found that by using a catalyst comprising molybdenum, vanadium, niobium and gold in combination with oxygen and in the absence of palladium, and wherein the molybdenum, vanadium, niobium and gold are present in specific amounts, ethane and/or ethylene may be oxidized to acetic acid with increased selectivity to acetic acid. Furthermore, it has been found possible using the catalysts of the present invention, to achieve a high selectivity to acetic acid with reduced selectivity to ethylene.

Accordingly, the present invention provides a catalyst composition for the oxidation of ethane and/or ethylene to acetic acid, which composition comprises in combination with oxygen the elements molybdenum, vanadium, niobium and gold in the absence of palladium according to the empirical formula : $Mo_aW_bAu_cV_dNb_eY_f$ (I)

wherein Y is one or more elements selected from the group consisting of : Cr, Mn, Ta, Ti, B, Al, Ga, In, Pt, Zn, Cd, Bi, Ce, Co, Rh, Ir, Cu, Ag, Fe, Ru, Os, K, Rb, Cs, Mg, Ca, Sr, Ba, Zr, Hf, Ni, P, Pb, Sb, Si, Sn, Tl, U, Re, Te and La;

a, b, c, d, e and f represent the gram atom ratios of the elements such that:

$0 < a \le 1$; $0 \le b < 1$ and $a + b = 1$;
$10^{-5} < c \le 0.02$;
$0.4 \le d \le 0.865$; $0.135 \le e \le 0.23$; and $0.55 \le d + e \le 1$;
$0 \le f \le 2$.

Catalysts embraced within the formula (I) include:-

$Mo_aW_bAu_cV_dNb_eY_f$
$Mo_aAu_cV_dNb_eY_f$
$Mo_aW_bAu_cV_dNb_e$
$Mo_aAu_cV_dNb_e$

Examples of suitable catalysts having the formula (I) include:-$Mo_{1.00}V_{0.455}Nb_{0.200}.AU_{0.0008}Oy$; $Mo_{1.00}V_{0.54}Nb_{0.163}Au_{0.0009}O_y$ and $Mo_{1.000}V_{0.661}Nb_{0.174}Au_{0.0009}O_y$ wherein y is a number which satisfies the valencies of the elements in the composition for oxygen.

Preferably, a > 0.01, and most preferably a = 1.

Preferably, c > 0.0001, and most preferably c > 0.0005. Preferably, c ≤ 0.002, and most preferably c ≤ 0.001.

Preferably, d ≥ 0.425, such as d ≥ 0.45, and, most preferably d ≥ 0.5. Preferably, d ≤ 0.8, and most preferably d ≤ 0.7.

Preferably, e ≥ 0.14, and most preferably, e ≥ 0.15. Preferably, e ≤ 0.20, and most preferably e ≤ 0.18.

Preferably d + e ≥ 0.6, such as d + e ≥ 0.7. Most preferably d + e ≥ 0.8. Preferably d + e ≤ 0.95, more preferably d + e ≤ 0.9.

Preferably, f ≤ 0.2, and most preferably f ≤ 0.02.

In a preferred embodiment, a > 0.01, 0.0001 < c ≤ 0.002, 0.425 ≤ d ≤ 0.8, 0.14 ≤ e ≤ 0.20, 0.6 ≤ d + e ≤ 0.95, and f ≤ 0.2; more preferably wherein 0.0005 < c ≤ 0.001, 0.45 ≤ d ≤ 0.7, e ≥ 0.15, d + e ≤ 0.9, and f ≤ 0.02; especially wherein d ≥ 0.5, e ≤ 0.18 and d + e ≥ 0.7, such as d + e ≥ 0.8. In this preferred embodiment, most preferably a = 1.

Y, when present, is preferably selected from the group consisting of Sn, Sb, Cu, Pt, Ag, Fe and Re.

An advantage of catalyst compositions according to the present invention is that they can be more active and selective in converting ethane and/or ethylene to acetic acid than compositions not according to the present invention. Typically, using the catalyst compositions of the present invention, a selectivity to acetic acid of at least 55mol% may be achieved. More preferably the selectivity to acetic acid that may be achieved is greater than 60%, such as greater than 70%.

In particular, using the catalyst compositions of the present invention, a high selectivity to acetic acid may be achieved in combination with a low, if any, selectivity to ethylene.

Typically, using the catalyst compositions of the present invention, the selectivity to ethylene is less than 30 mol%, preferably less than 20 mol%, and most preferably less than 10mol%.

Preferably, using the catalyst compositions of the present invention, the selectivity to acetic acid is at least 70mol% and the selectivity to ethylene is less than 10 mol%.

As used herein, selectivity refers to a percentage that reflects the amount of desired acetic acid product produced as compared to the total carbon in the products formed :-

$$\% \ selectivity \ = \ 100 * Moles \ of \ acetic \ acid \ produced \ / \ S$$

wherein S = the molar acid-equivalent sum (carbon basis) of all carbon-containing products, excluding the alkane in the effluent

The catalyst compositions may be prepared by any of the methods conventionally employed for the preparation of catalysts. Suitably the catalyst may be prepared from a solution of soluble compounds and/or complexes and/or compounds of each of the metals. The solution is preferably an aqueous system having a pH in the range from 1 to 12, preferably from 2 to 8, at a temperature of from 20° to 100°C.

Generally, a mixture of compounds containing the elements is prepared by dissolving sufficient quantities of soluble compounds and dispersing any insoluble compounds so as to provide a desired gram-atom ratio of the elements in the catalyst composition. The catalyst composition may then be prepared by removing the solvent from the mixture. The catalyst may be calcined by heating to a temperature of from 200 to 550°C, suitably in air or oxygen, for a period of from 1 minute to 24 hours. Preferably, the air or oxygen is slowly flowing.

The catalyst may be used unsupported or supported. Suitable supports include silica, alumina, zirconia, titania, silicon carbide and mixtures of two or more thereof.

Further details of a suitable method for preparing a catalyst composition may be found in, for example, EP-A-0166438.

The catalyst may be used in the form of a fixed or a fluidised bed.

In another embodiment the present invention provides a process for the selective production of acetic acid from a gaseous mixture comprising ethane and/or ethylene which process comprises contacting the gaseous mixture with a molecular oxygencontaining gas at elevated temperature in the presence of a catalyst composition as hereinbefore described.

The feed gas comprises ethane and/or ethylene, preferably ethane.

Ethane and/or ethylene may each be used in substantially pure form or admixed with one or more of nitrogen, methane, carbon dioxide and water in the form of steam, which may be present in major amounts, for example greater than 5 volume percent, or one or more of hydrogen, carbon monoxide, $C_3/C_4$ alkenes and alkenes, which may be present in minor amounts, for example less than 5 volume percent.

The molecular oxygen-containing gas may be air or a gas richer or poorer in molecular oxygen than air, for example oxygen. A suitable gas may be, for example, oxygen diluted with a suitable diluent, for example nitrogen.

It is preferred to feed, in addition to ethane and/or ethylene and the molecular oxygen-containing gas, water (steam) because this can improve the selectivity to acetic acid.

The elevated temperature may suitably be in the range from 200 to 500°C, preferably from 200 to 400°C.

The pressure may suitably be atmospheric or superatmospheric, for example in the range from 1 to 50 bar, preferably from 1 to 30 bar.

The catalyst composition is preferably calcined before use in the process of the invention. Calcination may suitably be achieved by heating at a temperature suitably in the range from 250 to 500°C in the presence of an oxygen-containing gas, for example air.

Operating conditions and other information applicable to the performance of the invention may be found in the aforesaid prior art, for example US Patent No. 4,250,346.

The process of the invention will now be further illustrated by reference to the following Examples.

Catalyst Preparation

Examples according to the present invention

### Catalyst A ($Mo_{1.00}V_{0.455}Nb_{0.200}Au_{0.0008}O_y$)

A solution 'A' was prepared by dissolving 22.070 g of ammonium molybdate and 0.0369 g of ammonium gold chloride in 100 ml of distilled water at 70°C with stirring. A solution 'B' was prepared by dissolving 6.652 g of ammonium vanadate in 150 ml of distilled water at 70°C with stirring. A solution 'C' was prepared by dissolving 6.704 g of niobium pentachloride and 7.821 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.00}V_{0.455}Nb_{0.200}Au_{0.0008}O_y.$$

### Catalyst B ($Mo_{1.00}V_{0.547}Nb_{0.163}Au_{0.0009}O_y$)

A solution 'A' was prepared by dissolving 22.070 g of ammonium molybdate and 0.0359 g of ammonium gold chloride in 100 ml of distilled water at 70°C with stirring. A solution 'B' was prepared by dissolving 6.555 g of ammonium vanadate in 150 ml of distilled water at 70°C with stirring. A solution 'C' was prepared by dissolving 5.134 g of niobium pentachloride and 5.992 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.00}V_{0.547}Nb_{0.163}Au_{0.0009}O_y.$$

### Catalyst C ($Mo_{1.00}V_{0.661}Nb_{0.174}Au_{0.00}O_y$)

A solution 'A' was prepared by dissolving 22.070 g of ammonium molybdate and 0.0382 g of ammonium gold chloride in 100 ml of distilled water at 70°C with stirring. A solution 'B' was prepared by dissolving '8.005 g of ammonium vanadate in 150 ml of distilled water at 70°C with stirring. A solution 'C' was prepared by dissolving 5.488 g of niobium pentachloride and 6.404 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.000}V_{0.661}Nb_{0.17}Au_{0.0009}O_y.$$

## Examples not according to the invention

### Comparative Example 1. ($Mo_{1.00}V_{0.423}Nb_{0.115}Au_{0.0008}O_y$)

A solution 'A' was prepared by dissolving 22.070 g of ammonium molybdate and 0.0345 g of ammonium gold chloride in 100 ml of distilled water at 70°C with stirring. A solution 'B' was prepared by dissolving 6.220 g of ammonium vanadate in 150 ml of distilled water at 70°C with stirring. A solution 'C' was prepared by dissolving 3.918 g of niobium pentachloride and 4.570 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.000}V_{0.423}Nb_{0.115}Au_{0.0008}O_y.$$

### Comparative Example 2. ($Mo_{1.00}V_{0.529}Nb_{0.124}Au_{0.0008}O_y$)

A solution 'A' was prepared by dissolving 22.070 g of ammonium molybdate and 0.0411 g of ammonium gold chloride in 100 ml of distilled water at 70°C with stirring. A solution 'B' was prepared by dissolving 7.741 g of ammonium vanadate in 150 ml of distilled water at 70°C with stirring. A solution 'C' was prepared by dissolving 4.189 g of niobium pentachloride and 4.889 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.000}V_{0.529}Nb_{0.124}Au_{0.0008}O_y.$$

### Comparative Example 3. ($Mo_{1.00}V_{0.638}Nb_{0.133}Au_{0.0009}O_y$)

A solution 'A' was prepared by dissolving 22.070 g of ammonium molybdate and 0.0395 g of ammonium gold chloride in 100 ml of distilled water at 70°C with stirring. A solution 'B' was prepared by dissolving 9.356 g of ammonium vanadate in 150 ml of distilled water at 70°C with stirring. A solution 'C' was prepared by dissolving 4.487 g of niobium pentachloride and 5.234 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.000}V_{0.638}Nb_{0.133}Au_{0.0009}O_y.$$

### Comparative Example 4. ($Mo_{1.00}V_{0.362}Nb_{0.143}Au_{0.0008}O_y$)

A solution 'A' was prepared by dissolving 22.070 g of ammonium molybdate and 0.0336 g of ammonium gold chloride in 100 ml of distilled water at 70°C with stirring. A solution 'B' was prepared by dissolving 5.281 g of ammonium vanadate in 150 ml of distilled water at 70°C with stirring. A solution 'C' was prepared by dissolving 4.828 g of niobium pentachloride and 5.632 g of oxalic acid in 100 ml of distilled water at 70°C with stirring. Each of the solutions A, B and C was left for 15 minutes to allow maximum solubilisation of the components. Solution C was then added to solution B rapidly with stirring at 70°C. The mixed solution B/C was stirred for 15 minutes at 70°C then added rapidly to solution A. The final mixed solution A/B/C was left to stir at 70°C for a further 15 minutes, after which the solution was heated to boiling to facilitate evaporation of the water. Full evaporation of the reactant mixture was achieved in 1.5 hours, resulting in a dry paste. The beaker with the dried paste was then transferred to an oven for further drying at 120°C for 2 hours. After drying, the catalyst precursor was ground to a fine powder and then sieved through a 0.2 mm mesh sieve. The resulting powdered catalyst cake was then calcined in static air in an oven at 400°C for 4 hours. The nominal formula of the oxide catalyst obtained was:

$$Mo_{1.000}V_{0.362}Nb_{0.143}Au_{0.0008}O_y.$$

### General Ethane Oxidation Reaction Method

Typically 5 ml of a powdered catalyst was mixed with 15 ml of glass beads of diameter 0.4 mm to form a diluted catalyst bed of 20 ml in volume. The diluted catalyst was then loaded into a fixed bed reactor made of Hastelloy of dimensions 12 mm internal diameter and length 40 cm: The catalyst was maintained in position in the centre of the reactor using quartz wall plugs with inert packing material above and below the catalyst bed. The apparatus was then pressure-tested at 20 bar with helium to check for leaks. The catalyst was then activated by heating to 220°C at 5°C/min in helium at 16 bar for 1 hour, to ensure full decomposition of catalyst precursors.

The required flows of ethane, ethylene, 20 % oxygen in helium and water were then introduced to the reactor, to ensure the required inlet composition. This composition was 52 % v/v ethane, 6.6 % v/v oxygen, 10 % v/v ethylene, 5 % v/v water and balance helium. The total feed flow rate was maintained at a level to ensure a feed GHSV of 3200/h. After equilibrating for 60 minutes, gas samples were taken from the outlet stream to a GC system (model Unicam 4400) to quantify ethane, ethylene, oxygen and helium.

The reactor temperature was maintained at 300°C for each of the catalysts A-C, in order to facilitate direct comparison. Following a further equilibration period of 60 minutes, liquid product collection was commenced and continued for a period of typically 18 hours. During the run period, the effluent gas composition was measured using GC analysis (ProGC, Unicam). Exit gas volume was measured over the run period by a water-gas meter. The liquid products were collected and weighed after the run period. Composition of the liquid products was measured using gas chromatography analysis (Unicam 4400 and 4200 fitted with TCD and FID detectors respectively).

From analysis of the feed and product flow rates and compositions the following parameters were calculated: .

### Conversions:

$$\text{of ethane} = (\text{inlet mol ethane - outlet mol ethane}) / \text{inlet mol ethane} * 100$$

$$\text{of oxygen} = (\text{inlet mol oxygen} - \text{outlet mol oxygen})/\text{inlet mol oxygen} * 100$$

.

**Selectivities:**

to acetic acid (C-mol %) = (outlet mol acetic acid * 2) / ((outlet mol ethylene * 2 - inlet mol ethylene * 2) + outlet mol CO + outlet mol $CO_2$ + outlet mol acetic acid * 2) * 100

to ethylene(C-mol %) = (outlet mol ethylene * 2)/ ((outlet mol ethylene * 2 - inlet mol ethylene * 2) + outlet mol CO + outlet mol $CO_2$ + outlet mol acetic acid * 2) * 100 to CO (C-mol %) = (outlet mol CO) / ((outlet mol ethylene * 2 - inlet mol ethylene * 2) + outlet mol CO + outlet mol $CO_2$ + outlet mol acetic acid * 2)* 100 to $CO_2$ (C-mol %) = (outlet mol $CO_2$) / ((outlet mol ethylene * 2 - inlet mol ethylene * 2) + outlet mol CO + outlet mol $CO_2$ + outlet mol acetic acid * 2) * 100 to $CO_x$ = selectivity to CO (C-mol %) + selectivity to $CO_2$ (C-mol %) STY (space time yield)% = (g acetic acid) /kg catalyst bed /hour

Typically, mass balance and carbon balance for a reaction was found to be 100 +/- 5 %.

**Experiments A to C and Comparative Examples 1-3**

Each catalyst was employed in the general reaction method described above. The results are given in Table I. Each catalyst was evaluated under standard conditions indicated.

Table I

| Catalyst | Conversion (%) | Selectivity (C-mol %) | | | | |
|---|---|---|---|---|---|---|
| | Ethane | Ethylene | AcOH | CO | $CO_2$ | $CO_x$ |
| 1 (comparative) | 7.3 | 29.9 | 54.1 | 10.6 | 5.5 | 16.1 |
| 2 (comparative) | 8.9 | 32.7 | 51.8 | 10.2 | 5.3 | 15.5 |
| 3 (comparative) | 4.5 | 31.6 | 51.3 | 13.6 | 3.6 | 17.2 |
| 4 (comparative) | 7.2 | 38.2 | 48.8 | 9.6 | 3.4 | 13.0 |
| A | 3.4 | 3.7 | 68.5 | 16.8 | 11.1 | 27.9 |
| B | 4.6 | 9.0 | 74.5 | 12.3 | 4.2 | 16.6 |
| C | 4.1 | 0.0 | 80.8 | 14.6 | 4.6 | 19.2 |

Conditions :

52 % v/v ethane, 6.6 % v/v oxygen, 10 % v/v ethylene, 5 % v/v water, balance helium, Temperature = 300°C, GHSV = 3200 $h^{-1}$, 16 barg.

The results in Table I indicate that for Mo-V-Nb-Au catalysts according to the present invention, when compared to the Comparative Examples, the selectivity profile unexpectedly changes towards the production of AcOH at the expense of ethylene.

**Claims**

1. A catalyst composition for the oxidation of ethane and/or ethylene to acetic acid, which composition comprises in combination with oxygen the elements molybdenum, vanadium, niobium and gold in the absence of palladium according to the empirical formula : $Mo_aW_bAu_cV_dNb_eY_f$ (I),
wherein Y is one or more elements selected from the group consisting of : Cr, Mn, Ta, Ti, B, Al, Ga, In, Pt, Zn, Cd, Bi, Ce, Co, Rh, Ir, Cu, Ag, Fe, Ru, Os, K, Rb, Cs, Mg, Ca, Sr, Ba, Zr, Hf, Ni, P, Pb, Sb, Si, Sn, Tl, U, Re, Te and La; and
a, b, c, d, e and f represent the gram atom ratios of the elements such that :

$0 < a \leq 1$; $0 \leq b < 1$ and $a + b = 1$;

$10^{-5} < c \leq 0.02$;
$0.4 \leq d \leq 0.865$; $0.135 \leq 0.23$; and $0.55 \leq d + e \leq 1$; and
$0 \leq f \leq 2$.

2. A catalyst composition as claimed in claim 1, selected from the group consisting of: $Mo_aW_bAu_cV_dNb_eY_f$; $Mo_a.Au_cV_d\text{-}Nb_eY_f$; $Mo_aW_b.Au_cV_dNb_e$ and $Mo_a.Au_cV_dNb_e$.

3. A catalyst composition as claimed in claim 1 or claim 2, wherein $a > 0.01$, $0.0001 < c \leq 0.002$, $0.425 \leq d \leq 0.8$, $0.14 \leq e \leq 0.20$, $0.6 \leq d + e \leq 0.95$, and $f \leq 0.2$.

4. A catalyst composition as claimed in claim 3, wherein $0.0005 < c \leq 0.001$, $0.45 \leq d \leq 0.7$, $e \geq 0.15$, $d + e \leq 0.9$, and $f \leq 0.02$.

5. A catalyst composition as claimed in claim 4, wherein $d \geq 0.5$, $e \leq 0.18$, and $d + e \geq 0.7$.

6. A catalyst composition as claimed in claim 5, wherein $d + e \geq 0.8$.

7. A catalyst composition as claimed in any one of the preceding claims, wherein $a = 1$.

8. A catalyst composition as claimed in any one of the preceding claims, wherein Y is selected from the group consisting of Sn, Sb, Cu, Pt, Ag, Fe and Re.

9. A catalyst composition as claimed in claim 1 having the formula selected from the group consisting of: $Mo_{1.00}V_{0.455}Nb_{0.200}.Au_{0.0008}O_y$; $Mo_{1.00}V_{0.547}Nb_{0.163}Au_{0.0009}O_y$ and $Mo_{1.000}V_{0.661}Nb_{0.174}Au_{0.009}O_y$ wherein y is a number which satisfies the valencies of the elements in the composition for oxygen.

10. A process for the selective production of acetic acid from a gaseous mixture comprising ethane and/or ethylene which process comprises contacting the gaseous mixture with a molecular oxygen-containing gas at elevated temperature in the presence of a catalyst composition as claimed in any one of the preceding claims.

11. A process as claimed in claim 10 in which the catalyst is used in the form of a fluidized bed.

**Patentansprüche**

1. Katalysatorzusammensetzung für die Oxidation von Ethan und/oder Ethylen zu Essigsäure, wobei die Zusammensetzung in Kombination mit Sauerstoff die Elemente Molybdän, Vanadium, Niob und Gold in Abwesenheit von Palladium gemäß der empirischen Formel:

$Mo_aW_bAu_cV_dNb_cY_f$(I) umfasst,
wobei Y ein oder mehrere Elemente, ausgewählt aus der Gruppe, bestehend aus: Cr, Mn, Ta, Ti, B, Al. Ga, In, Pt, Zn, Cd, Bi, Ce, Co, Rh, Ir, Cu, Ag, Fe, Ru, Os, K, Rb, Cs, Mg, Ca, Sr, Ba, Zr, Hf, Ni, P, Pb, Sb, Si. Sn, Tl, U, Re, Te und La, ist; und
a, b, c, d, e und f die Grammatom-Verhältnisse der Elemente darstellen, so dass:

$0 < a \leq 1$; $0 \leq b < 1$ 1 und $a + b = 1$:
$10^{-5} < c$ 0,02;
$0,4 \leq d \leq 0,865$; $0,135 \leq e \leq 0,23$; und $0,55 \leq d + e \leq 1$; und
$0 \leq f \leq 2$.

2. Katalysatorzusammensetzung nach Anspruch 1, ausgewählt aus der Gruppe, bestehend aus:

$Mo_aW_bAu_cV_dNb_cY_f$; $Mo_aAu_cV_dNb_eY_f$; $Mo_AW_bAu_cV_dNb_e$ und $Mo_aAu_cV_dNb_e$.

3. Katalysatorzusammensetzung nach Anspruch 1 oder Anspruch 2, wobei $a > 0,01$, $0,0001 < c$ 0,002, $0,425$ s $d \leq 0,8$, $0,14 \leq e \leq 0,20$, $0,6 \leq d + e \leq 0,95$, und $f \leq 0,2$.

4. Katalysatorzusammensetzung nach Anspruch 3, wobei $0,0005 < c \leq 0,001$, $0,45 \leq d \leq 0,7$, $e \geq 0,15$, $d + e \leq 0,9$,

und f ≤ 0,02.

5. Katalysatorzusammensetzung nach Anspruch 4, wobei d ≥ 0.5, e ≤ 0,18, und d + e ≥ 0,7.

6. Katalysatorzusammensetzung nach Anspruch 5, wobei d + e ≥ 0,8.

7. Katalysatorzusammensetzung nach einem der vorstehenden Ansprüche, wobei a = 1.

8. Katalysatorzusammensetzung nach einem der vorstehenden Ansprüche, wobei Y aus der Gruppe ausgewählt ist, bestehend aus Sn, Sb. Cu, Pt, Ag, Fe und Re.

9. Katalysatorzusammensetzung nach Anspruch 1 mit der Formel, ausgewählt aus der Gruppe, bestehend aus $Mo_{1.00}V_{0.455}Nb_{0.200}Au_{0.0008}O_y$; $Mo_{1.00}V_{0.547}Nb_{0.163}Au_{0.0009}O_y$ und $Mo_{1.000}V_{0.661}Nb_{0.174}Au_{0.0009}O_y$, wobei y eine Zahl ist, die den Valenzen der Elemente in der Zusammensetzung für Sauerstoff entspricht.

10. Verfahren zur selektiven Herstellung von Essigsäure aus einem Gasgemisch, umfassend Ethan und/oder Ethylen, wobei das Verfahren das Kontaktieren des Gasgemisches mit einem molekularen Sauerstoff enthaltenden Gas bei erhöhter Temperatur in Gegenwart einer Katalysatorzusammensetzung nach einem der vorstehenden Ansprüche umfasst.

11. Verfahren nach Anspruch 10, bei dem der Katalysator in Form eines Fließbettes verwendet wird.

## Revendications

1. Composition de catalyseur pour l'oxydation de l'éthane et/ou de l'éthylène en acide acétique, laquelle composition comprend en association avec l'oxygène les éléments molybdène, vanadium, niobium et or en l'absence de palladium selon la formule empirique :

$Mo_aW_bAu_cV_dNb_eY_f$ (I),
dans laquelle Y représente un ou plusieurs éléments choisis dans le groupe constitué par : le Cr, le Mn, le Ta, le Ti, le B, l'Al, le Ga, l'In, le Pt, le Zn, le Cd, le Bi, le Ce, le Co, le Rh, l'Ir, le Cu, l'Ag, le Fe, le Ru, l'Os, le K, le Rb, le Cs, le Mg, le Ca, le Sr, le Ba, le Zr, le Hf, le Ni, le P, le Pb, le Sb, le Si, le Sn, le Tl, l'U, le Re, le Te et le La ; et a, b, c, d, e et f représentent les rapports atomiques en gramme des éléments tels que :

$0 < a ≤ 1$; $0 ≤ b < 1$ et $a + b = 1$;
$10^{-5} < c ≤ 0,02$ ;
$0,4 ≤ d ≤ 0,865$; $0,135 ≤ e ≤ 0,23$; et $0,55 ≤ d + e ≤ 1$ ; set
$0 ≤ f ≤ 2$.

2. Composition de catalyseur selon la revendication 1, choisie dans le groupe constitué par : le $Mo_aW_bAu_cV_dNb_eY_f$; le $Mo_aAu_cV_dNb_eY_f$ ; le $Mo_aW_bAu_cV_dNb_e$ et le $Mo_aAu_cV_dNb_e$.

3. Composition de catalyseur selon la revendication 1 ou la revendication 2, dans laquelle a > 0,01, 0,0001 < c ≤ 0,002, 0,425 ≤ d ≤ 0,8, 0,14 ≤ e ≤ 0,20, 0,6 ≤ d + e ≤ 0,95, et f ≤ 0.2.

4. Composition de catalyseur selon la revendication 3, dans laquelle 0,0005 < c ≤ 0,001, 0,45 ≤ d ≤ 0,7, e ≤ 0,15, d + e ≤ 0,9, et f ≤ 0,02.

5. Composition de catalyseur selon la revendication 4, dans laquelle d ≥ 0,5, e ≤ 0,18, et d + e ≥ 0,7.

6. Composition de catalyseur selon la revendication 5, dans laquelle d + e ≥ 0,8.

7. Composition de catalyseur selon l'une quelconque des revendications précédentes, dans laquelle a = 1.

8. Composition de catalyseur selon l'une quelconque des revendications précédentes, dans laquelle Y est choisi dans le groupe constitué par le Sn, le Sb, le Cu, le Pt, l'Ag, le Fe et le Re.

9. Composition de catalyseur selon la revendication 1 répondant à une formule choisie dans le groupe constitué par : le $Mo_{1,00}V_{0,455}Nb_{0,200}Au_{0,0008}O_y$ ; le $Mo_{1,00}V_{0,547}Nb_{0,163}Au_{0,0009}O_y$ et le $Mo_{1,000}V_{0,661}Nb_{0,174}Au_{0,0009}O_y$ dans lesquels y représente un nombre qui satisfait les valences des éléments de la composition pour l'oxygène.

10. Procédé pour la production sélective d'acide acétique à partir d'un mélange gazeux comprenant de l'éthane et/ou de l'éthylène, lequel procédé comprend la mise en contact du mélange gazeux avec un gaz contenant de l'oxygène moléculaire à température élevée en présence d'une composition de catalyseur selon l'une quelconque des revendications précédentes.

11. Procédé selon la revendication 10 dans lequel le catalyseur est utilisé sous la forme d'un lit fluidisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4250346 A
- EP 1043064 A
- WO 9920592 A

- DE 19630832
- DE 19630832 A1
- EP 0166438 A